(19) Europäisches Patentamt
European Patent Office
Office européen des brevets

(11) EP 1 229 127 B1

(12) EUROPÄISCHE PATENTSCHRIFT

(45) Veröffentlichungstag und Bekanntmachung des Hinweises auf die Patenterteilung:
**14.09.2005 Patentblatt 2005/37**

(51) Int Cl.[7]: **C12P 41/00**

(21) Anmeldenummer: **02001124.3**

(22) Anmeldetag: **24.01.2002**

# (54) Verfahren zur enzymatischen Herstellung von enantiomerenreinen 1,3-Dioxolan-4-on- und 1,3-Oxathiolan-5-on-Derivaten

Method for the enzymatic preparation of enantiomerically pure derivatives of 1,3-Dioxolan-4-one and 1,3-Oxathiolan-5-one

Méthode pour la préparation des enantiomers pures des dérivés de 1,3-Dioxolan-4-one et de 1,3-Oxathiolan-5-one

(84) Benannte Vertragsstaaten:
**AT BE CH CY DE DK ES FI FR GB GR IE IT LI LU MC NL PT SE TR**

(30) Priorität: **31.01.2001 DE 10104231**

(43) Veröffentlichungstag der Anmeldung:
**07.08.2002 Patentblatt 2002/32**

(73) Patentinhaber: **Consortium für elektrochemische Industrie GmbH**
**81379 München (DE)**

(72) Erfinder:
- **Popp, Alfred, Dr.**
  **82008 Unterhaching (DE)**
- **Stohrer, Jürgen, Dr.**
  **82049 Pullach (DE)**
- **Petersen, Hermann, Dr.**
  **84489 Burghausen (DE)**
- **Gilch, Andrea**
  **85419 Mauern (DE)**
- **Rockinger-Mechlem, Jodoca**
  **82205 Gilching (DE)**

(74) Vertreter: **Potten, Holger et al**
**Wacker-Chemie GmbH**
**Zentralabteilung Patente,**
**Marken und Lizenzen**
**Hanns-Seidel-Platz 4**
**81737 München (DE)**

(56) Entgegenhaltungen:
**WO-A-00/22157** **WO-A-91/11186**

- **HOF R P ET AL: "Synthesis and lipase-catalysed resolution of 5-(hydroxymethyl)-1,3-dioxolan-4-ones: masked glycerol analogues as potential building blocks for pharmaceuticals" JOURNAL OF ORGANIC CHEMISTRY, AMERICAN CHEMICAL SOCIETY. EASTON, US, Bd. 61, Nr. 10, 17. Mai 1996 (1996-05-17), Seiten 3423-3427, XP002163350 ISSN: 0022-3263**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur enzymatischen Herstellung von enantiomerenreinen 1,3-Dioxolan-4-on- und 1,3-Oxathiolan-5-on-Derivaten.

Enantiomerenreine Derivate dienen als Ausgangsmaterialien bzw. Zwischenprodukte bei der Synthese von Agrochemikalien und Pharmazeutika. Viele dieser Verbindungen werden zur Zeit als Racemat oder Diastereomerengemisch hergestellt und vermarktet. In vielen Fällen wird der gewünschte physiologische Effekt aber nur von einem Enantiomer/ Diastereomer bewirkt. Das andere Isomer ist im günstigsten Fall inaktiv, es kann aber auch dem gewünschten Effekt entgegenwirken oder sogar toxisch sein. Verfahren zur Trennung von Racematen werden deshalb immer wichtiger für die Darstellung hochenantiomerenreiner Verbindungen.

Es ist bekannt, dass die Racemattrennung chiraler Verbindungen mit Hilfe von Enzymen durchgeführt werden kann. In einer Vielzahl von Publikationen werden enzymatisch kinetische Racematspaltungen von Estern mit Lipasen und Esterasen beschrieben. Es gibt jedoch bisher kein Verfahren, dass die einfache Trennung von 1,3-Dioxolan-4-on- bzw. 1,3-Oxathiolan-5-on-Derivaten erlaubt. Enantiomerenreine 1,3-Dioxolan-4-one sind von großem Interesse für die Herstellung von anti-viral wirksamen Verbindungen, wie z. B. das 1,3-Dioxolanyl-Nucleosid "Dioxolane-T" (NB = Thymin in Gleichung 1) und ähnlichen Strukturen (*Bioorg. Med. Chem. Lett.* 1993, 3(2), S. 169-174).

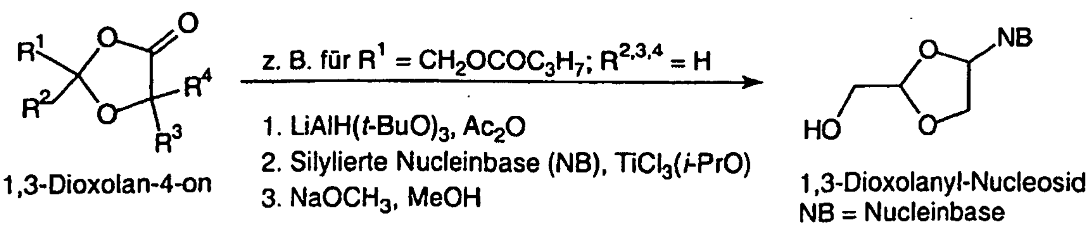


Gleichung 1

Zur Herstellung von enantiomerenreinen 1,3-Dioxolanyl-Nucleosiden wird die Trennung in die Enantiomere bisher auf der erheblich teureren Nucleosidstufe durchgeführt. Erstmals beschrieben wird dieses Verfahren von L. J. Wilson et al. (*Bioorg. Med. Chem. Lett.* 1993, 3(2), S. 169-174). Der Buttersäureester der primären Hydroxylgruppe eines 1,3-Dioxolanyl-Nucleosids wird dort mit Hilfe von Schweineleberesterase hydrolysiert und so die beiden reinen Enantiomere in guten optischen Ausbeuten erhalten. WO 00/22157 (Erfinder: Yao, Y. et al.) beschreibt eine Variante dieses Verfahrens durch Resolution in nicht-homogenen Systemen.
Enantiomerenreine 1,3-Oxathiolan-5-one sind ebenso von großem Interesse für die Herstellung von anti-viral wirksamen Verbindungen, wie z. B. das 1,3-Oxathiolanyl-Nucleosid Coviracil® (auch Emtricitabine, früher FTC, 4-Amino-5-fluoro-1-[(2R,5S)-2-(hydroxymethyl)-1,3-oxathiolan-5-yl]-2(1H)-pyrimidinone; Gleichung 2) und ähnlichen Strukturen (*J. Org. Chem.* 1992, 57(21), S. 5563-5565, WO 91/11186, WO 92/14743, WO 00/22157).

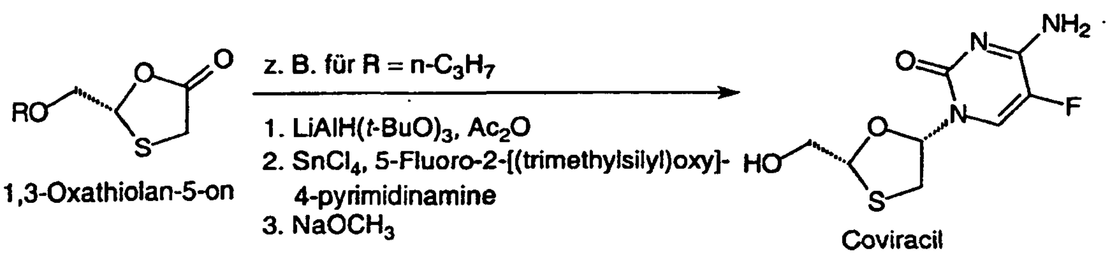


Gleichung 2

Zur Herstellung von enantiomerenreinen 1,3-Oxathiolanyl-Nucleosiden kann die Trennung in die Enantiomere auf verschiedenen Stufen erfolgen. So ist eine enzymatische Racematspaltung auf der Oxathiolanonstufe möglich. Diese wird von Liotta et al. (WO 91/11186) beschrieben. Dabei wird die Stereoselektion durch eine enzymatische Esterspaltung eines Substituenten in 2-Position des Oxathiolanrings erreicht (Gleichung 3).

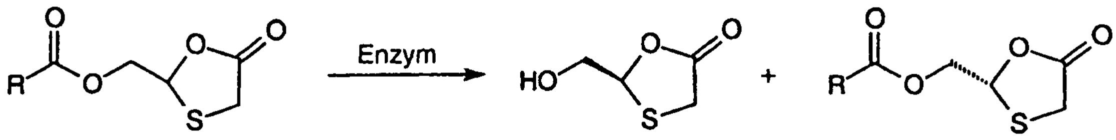


Gleichung 3

Als Beispiel wird die Hydrolyse des Buttersäureesters (R = $C_3H_7$) in Gegenwart von Schweineleberesterase (PLE) genannt.

Die zweite Möglichkeit besteht in der Racematspaltung auf der erheblich teureren Nucleosidstufe. Beschrieben wird dieses Verfahren von Liotta et al. (*J. Org. Chem.* 1992, 57(21), S. 5563-5565 und WO 92/14743). Dabei werden verschiedene Esteracylgruppen des Nucleosidracemats in Gegenwart von Lipasen bzw. Proteasen stereoselektiv abgespalten (Gleichung 4).

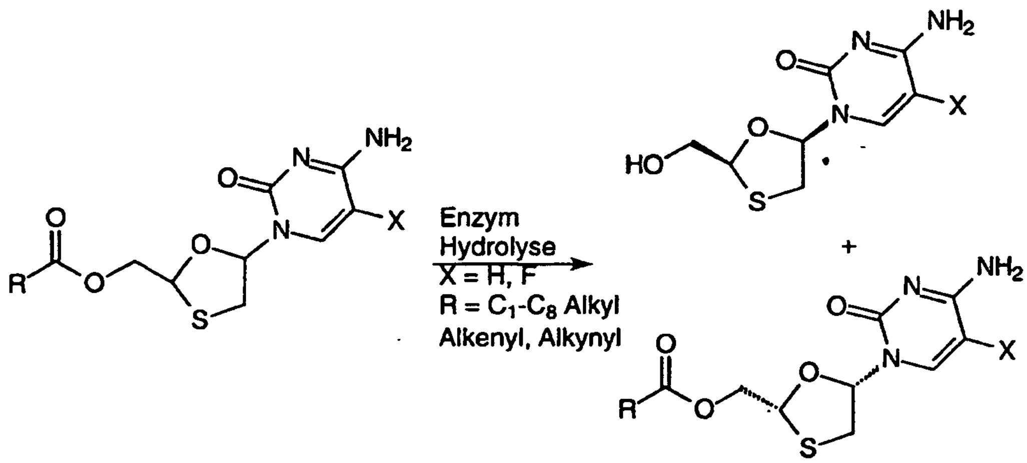


Gleichung 4

Dabei werden z. T. hohe Enantiomerenüberschüsse (ee(Ester) > 98%)bei guten Ausbeuten (y(Ester) - 45%) erreicht.
Eine Verbesserung des Verfahrens aus WO 92/14743 findet sich in WO 00/22157 (Erfinder: Yao, Y. et al.) durch die Verwendung nicht-homogener Reaktionssysteme (Zusatz von nicht-wassermischbaren Cosolventien) zur Racematspaltung von Oxathiolanyl-Nucleosiden.
Diese Verfahren, die Racematspaltung auf einer sehr späten Stufe durchzuführen, bergen den gravierenden Nachteil eines unnötigen Materialverbrauchs und hohen Anlagenbelegungszeiten, da die maximale Ausbeute einer Racematspaltung bei 50% liegt.

Die restlichen 50% (die Verbindung mit der falschen Händigkeit) werden im Regelfall verworfen.

Aufgabe der vorliegenden Erfindung ist es, ein Verfahren zur Herstellung von enantiomerenreinen 1,3-Dioxolan-4-on- und 1,3-Oxathiolan-5-on-Derivaten zur Verfügung zu stellen, welches kostengünstig ist und die genannten Nachteile vermeidet.

Die Aufgabe wird gelöst durch ein Verfahren, bei dem ein Gemisch enthaltend enantiomere 1,3-Dioxolan-4-on- bzw. 1,3-Oxathiolan-5-on-Derivate und ein hydrolytisch wirksames Enzym in Gegenwart eines Nucleophils in Kontakt gebracht werden, wobei der Dioxolanon- bzw. Oxathiolanonring eines Enantiomers durch das hydrolytisch wirksame Enzym gespalten wird und nach erfolgter Spaltung des einen Enantiomers das nichtgespaltene Enantiomer des 1,3-Dioxolan-4-on- bzw. 1,3-Oxathiolan-5-on-Derivats isoliert wird.

Das erfindungsgemäße Verfahren trennt ein Enantiomerengemisch auf der Dioxolanon- bzw. Oxathiolanonstufe und stellt so ein enantiomerenreines Derivat zu Verfügung, welches nun in an sich bekannter Weise die Herstellung eines enantiomerenreinen 1,3-Dioxolanyl- oder 1,3-Oxathiolanyl-Nucleosids ermöglicht.

1,3-Dioxolan-4-one bzw. 1,3-Oxathiolan-5-one besitzen im Ring eine hydrolytisch labile Esterbindung, die durch eine enzymkatalysierte Reaktion gespalten werden kann. Überraschend wurde gefunden, dass sich diese Esterbindung im Dioxolanon- bzw. Oxathiolanonring sowohl mit hoher Enantioselektivität, als auch hoher Regioselektivität gegenüber anderen in der Verbindung vorhandenen hydrolytisch labilen Gruppen durch ein hydrolytisch wirksames Enzym spalten lässt.

Vorzugsweise ist das erfindungsgemäße Verfahren daher dadurch gekennzeichnet, dass ein Gemisch enthaltend enantiomere 1,3-Dioxolan-4-on- bzw. 1,3-Oxathiolan-5-on-Derivate mit einem Enzym, das zur Spaltung einer Esterbindung befähigt ist in Gegenwart eines Nucleophils der allgemeinen Formel NuH in Kontakt bringt, so dass bevorzugt ein Enantiomer gespalten wird.

Diese Spaltung ist schematisch in Gleichung 5 dargestellt,

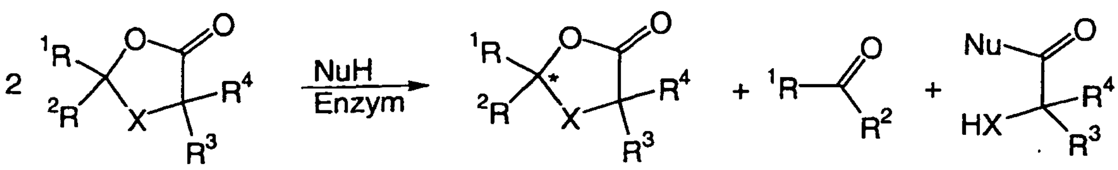


Enantiomerengemisch Reinenantiomer

Gleichung 5

wobei X = Sauerstoff oder Schwefel und

die Reste $R^1$ und $R^2$ ungleich sind und unabhängig voneinander ausgewählt sind aus der Gruppe H, substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl, $CR^8R^9$-$O_n$-$(CO)_m$-$R^{10}$ und

die Reste $R^3$ und $R^4$ unabhängig voneinander ausgewählt sind aus der Gruppe H substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, , $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl oder die Reste $R^3$ und $R^4$ zusammen mit dem Kohlenstoff an den sie gebunden sind, ein unsubstituiertes oder substituiertes oder ein Heteroatom enthaltendes Cycloalkyliden bilden, und Nu $OR^5$, $SR^5$, oder $NR^6R^7$ bedeutet wobei

der Reste $R^5$ ausgewählt sind aus der Gruppe H, substituiertes oder unsubstituiertes $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl und die Reste $R^6$ und $R^7$ unabhängig voneinander ausgewählt sind aus der Gruppe H, substituiertes oder unsubstituiertes $C_1$-$C_{18}$-Alkyl, $C_2$-$C_{18}$-Alkenyl, $C_2$-$C_{18}$-Alkinyl, $C_6$-$C_{18}$-Aryl, $C_3$-$C_{18}$-Heteroaryl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl und

die Reste $R^8$ und $R^9$ unabhängig voneinander ausgewählt sind aus der Gruppe substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, , $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl oder

die Reste $R^8$ und $R^9$ zusammen mit dem Kohlenstoff an den sie gebunden sind, ein unsubstituiertes oder substituiertes oder ein Heteroatom enthaltendes Cycloalkyliden bilden, und

m und n unabhängig voneinander 0 oder 1 bedeuten, und für den Rest $R^{10}$ gilt:

wenn m = 0 dann ist Rest $R^{10}$ ausgewählt aus der Gruppe substituiertes oder unsubstituiertes, $C_1$-$C_{18}$-Alkyl, $C_2$-$C_{18}$-Alkenyl oder $C_2$-$C_{18}$-Alkinyl, substituiertes oder unsubstituiertes $C_6$-$C_{18}$-Aryl, $C_3$-$C_{18}$-Heteroaryl, substituiertes oder unsubstituiertes Silaalkyl oder Silaaryl, und

wenn m = 1 dann ist Rest $R^{10}$ ausgewählt aus der Gruppe substituiertes oder unsubstituiertes Aryl, substituiertes oder unsubstituiertes, $C_1$-$C_{18}$-Alkyl, $C_2$-$C_{18}$-Alkenyl oder $C_2$-$C_{18}$-Alkinyl.

Soweit es sich bei den Resten um substituierte Reste handelt, sind diese vorzugsweise durch Alkyl-, Alkenyl-, Alkinyl-, Aryl-, Heteroaryl-, Hydroxy, Alkoxy-, Carboxylat-, Alkoxycarbonyl-, Amino-, Nitro- oder Halogenreste substituiert.

Soweit die vorstehend genannten Reste ein Heteroatom enthalten, handelt es sich dabei vorzugsweise um O, N oder S.

Bevorzugt Verwendung finden Enantiomerengemische der allgemeinen Formel (I),

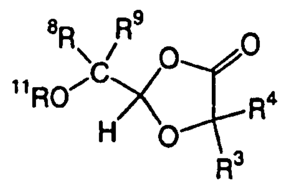

(I)

wobei $R^3$, $R^4$, $R^8$ und $R^9$ die bereits genannte Bedeutung haben und $R^{11}$ substituiertes oder unsubstituiertes, verzweigtes oder unverzweigtes $C_1$-$C_{18}$-Alkyl, $C_2$-$C_{18}$-Alkenyl oder $C_2$-$C_{18}$-Alkinyl, substituiertes oder unsubstituiertes Aryl, substituiertes oder unsubstituiertes Silaalkyl oder Silaaryl bedeutet oder $R^{11}$ $COR^{10}$ bedeutet, wobei $R^{10}$ die bereits genannte Bedeutung besitzt.

Besonders bevorzugt Verwendung finden Enantiomerengemische der allgemeinen Formel (II),

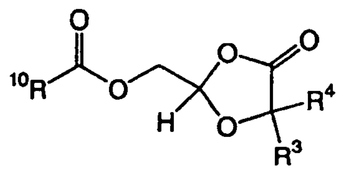

(II)

wobei $R^3$ und $R^4$ die bereits genannte Bedeutung besitzen und $R^{10}$ ausgewählt ist aus der Gruppe substituiertes oder unsubstituiertes Aryl, substituiertes oder unsubstituiertes, $C_1$-$C_{18}$-Alkyl, $C_2$-$C_{18}$-Alkenyl oder $C_2$-$C_{18}$-Alkinyl.

Das Nucleophil NuH ist bevorzugt ein sauerstoffhaltiges Nucleophil $OR^5$.

Besonders bevorzugt handelt es sich bei dem sauerstoffhaltigen Nucleophil um einen niederen, unverzweigten Alkohol, (z. B. Methanol ($R^5 = CH_3$) oder Ethanol ($R^5 = CH_2CH_3$)) oder Wasser ($R^5$ = H).

Für das erfindungsgemäße Verfahren sind prinzipiell alle Enzyme, die zur Spaltung einer Esterbindung befähigt sind, geeignet. Bevorzugt handelt es sich um eine Lipase oder Esterase der Klasse 3.1 gemäß Internationaler Enzym-Nomenklature, Committee of the International Union of Biochemistry and Molecular Biology. Wegen ihrer einfacheren Zugänglichkeit besonders bevorzugt handelt es sich um Lipasen oder Esterasen mikrobiellen Ursprungs, Schweinepankreaslipase, Pferdeleberesterase oder Schweineleberesterase.

Als Enzyme mikrobiellen Ursprungs seien beispielsweise genannt Enzyme aus Pilzen, Hefen oder Bakterien wie beispielsweise Alcaligenes sp., Aspergillus niger, Aspergillus oryzae, Bacillus sp., Bacillus stearothermophilus, Bacillus thermoglucosidasius, Candida antarctica, Candida lipolytica, Candida rugosa, Chromobacterium viscosum, Geotrichium candium, Mucor miehei, Penicillium camembertii, Penicillium roquefortii, Pseudomonas cepacia, Pseudomonas fluorescens, Pseudomonas sp., Rhizomucor javanicus, Rhizopus arrhizus, Rhizopus niveus, Saccharomyces cerevisae, Thermoanaerobium brockii, Thermomyces lanuginosa. Besonders bevorzugt werden dabei Lipasen und Esterasen aus Candida-Arten wie zum Beispiel Candida antarctica B.

Als Enzym ganz besonders bevorzugt sind Novozym® 435, 525 (käuflich erhältlich bei Firma Novo, Dänemark), Chirazyme® L2, E1, E2 und L7 (käuflich erhältlich bei Firma Böhringer Mannheim, Deutschland).

Das Enzym wird in der Reaktion direkt oder als Immobilisat gebunden an unterschiedlichste Träger eingesetzt.

Ein Immobilisat kann in an sich bekannter Weise hergestellt werden. Dies ist beispielsweise möglich durch Lösen des Enzyms in einem Puffer bei geeignetem pH und anschließender passiver Adsorption an den Träger wie z. B. Diatomeenerde (Celite®), Aktivkohle, Aluminiumoxid, Kieselgel, Kieselguhr, monodispers lösliche Organosiloxanpartikel oder Harze (z. B. Amberlite®, Dowex®). Alternativ können die Enzyme auch kovalent an den Träger gebunden werden (z. B. Polystyrol oder Epoxy-Harze wie Eupergit®). Ein beispielsweise derart an einen Träger gebundenes Enzym kann durch Lyophilisieren getrocknet werden.

Die im erfindungsgemäßen Verfahren einzusetzende Menge an Enzym hängt von der Art des Edukts, Produkts und der Aktivität der Enzympräparation ab. Die für die Reaktion optimale Enzymmenge kann durch einfache Vorversuche ermittelt werden.

Je nach Enzym liegt das Enzym-Substratverhältnis berechnet als Molverhältnis zwischen Enzym und Dioxolanon/Oxathiolanon-Derivat in der Regel zwischen 1 : 1000 und 1 : 50000000 oder mehr, bevorzugt bei 1 : 10000 bis 1 : 5000000.

Das erfindungsgemäße Verfahren kann sowohl in reinem Nucleophil (NuH) als Lösungsmittel als auch in Mischungen des Nucleophils (NuH) mit aprotischen oder protogenen Lösungsmitteln oder Lösungsmittelgemischen durchgeführt werden, sofern diese die Reaktivität des hydrolytisch wirksamen Enzyms nicht beeinflussen oder zu unerwünschten Nebenreaktionen führen.

Vorteilhafterweise wird die Reaktion in einer Mischung aus dem Nucleophil und einem geeigneten Lösungsmittel durchgeführt. Geeignete Lösungsmittel sind beispielsweise aliphatische oder aromatische Kohlenwasserstoffe wie Hexan, Cyclohexan, Petrolether oder Toluol, halogenierte Kohlenwasserstoffe wie Methylenchlorid oder Chloroform, Ether wie Methyl-*tert*.-Butylether (MTBE), Diethylether, Diisopropylether, THF oder Dioxan, Ester, Acetonitril oder gegebenenfalls Alkohole, die kein Nukleophil im Sinne o. g. enzymatischer Reaktion darstellen, wie z. B. tertiäre Alkohole, oder Mischungen der genannten Verbindungen.

Das Verhältnis Nucleophil/ Lösungsmittel (v/v) liegt dabei vorzugsweise in einem Bereich von 1 : 10000 bis 1000 : 1.

Besonders bevorzugt sind Mischungen des Nucleophils mit aprotische Lösungsmittel, wie MTBE oder Diisopropylether in einem Verhältnis Nucleophil/ Lösungsmittel (v/v) von 1 : 100 bis 100 : 1.

Wird als Nucleophil Wasser verwendet (Nu = OH), so kann, um einen vorgegebenen pH-Wert einzuhalten, dieser durch Zugabe eines Puffers eingestellt werden. Bevorzugt verwendet wird dazu ein $Na_2HPO_4$/ $NaH_2PO_4$-Puffer mit einem pH von 7,0. Zum selben Zweck kann auch eine wässrige Lauge, bevorzugt die Lösung eines Alkalihydroxyds in Wasser, besonders bevorzugt die wässrige Lösung von NaOH oder KOH, zudosiert werden.

Die Reaktion wird vorteilhafterweise bei einer Temperatur zwischen 0 °C und 75 °C durchgeführt, bevorzugt zwischen 10 °C und 60 °C, besonders bevorzugt zwischen 20 °C und 50 °C.

Die Reaktionszeiten betragen je nach Substitutionsmusters des Dioxolanons, Wahl des Nucleophils und Lösungsmittels und Enzymart und -menge zwischen 10 Minuten und 7 Tagen. Bevorzugt liegen die Reaktionszeiten zwischen 1 und 48 Stunden.

Der Reaktionsverlauf läßt sich leicht mit üblichen Methoden beispielsweise durch HPLC verfolgen. Bevorzugt kann die Bestimmung des Reaktionsverlaufs durch Messung der Änderung des optischen Drehwerts der Reaktionslösung in einem Polarimeter erfolgen. Besonders bevorzugt erfolgt die Bestimmung des Reaktionsverlaufs online, durch Messung des optischen Drehwerts im einem Nebenkreislauf des Reaktors. Die Reaktion kann je nach gewünschtem Ergebnis (hohe Umsetzung, hoher Enantiomerenüberschuß des Substrats) beendet werden. Im Idealfall ist die Reaktion bei einem Umsatz von 50 % bei einer hohen Enantiomerenreinheit im Substrat beendet.

Vorzugsweise wird die Reaktion beispielsweise durch Separation des Substrats bzw. des Produkts vom Enzym, z. B. durch Extraktion der wässrigen Phase oder Filtration beendet. Der Abbruch der Reaktion kann auch durch Desaktivierung des Enzyms, z. B. durch thermische oder chemische Denaturierung erfolgen.

Falls die Reaktion durch wiederholtes, kontinuierliches Pumpen der Reaktionslösung durch einen mit Enzym gefüllten Behälter durchgeführt wird, (eine besonders bevorzugte Verfahrensführung), wird die Reaktion vorzugsweise durch Beenden des Umpumpens beendet.

Die Isolierung des nichtgespaltenen reinen Enantiomers erfolgt vorzugsweise durch Abtrennung der bei der Reaktion entstandenen Nebenprodukte und des Lösungsmittels.

Die bei der Spaltung eines 1,3-Dioxolan-4-on- oder 1,3-Oxathiolan-5-onrings entstehende freie Carbonylverbindung $R^1COR^2$ und das Säurederivat $HXCR^3R^4CONu$ können aus der Reaktionslösung durch einfache physikalische Operationen abgetrennt werden. Vorzugsweise geschieht dies durch Destillation.

Darüber hinaus können weitere Abbauprodukte, die bei der Spaltung weiterer funktioneller Gruppen im Molekül gebildet werden leicht abgetrennt werden. Vorzugsweise geschieht dies durch Destillation.

Bevorzugt werden zunächst die niedrigsiedenden Verbindungen destillativ abgetrennt. Überraschend wurde gefunden, dass der Alkohol ($R^1$ = H, $R^2$ = $CH_2OH$), der als Nebenprodukt bei der Racematspaltung eines Esterdioxolanons (X = O; $R^1$ = H, $R^2$ = $CH_2$-O-(CO)-$R^{10}$) entsteht, durch einfache Extraktion, bevorzugt mit Wasser, abgetrennt werden kann.

Die bei der enzymatischen Reaktion entstehende Carbonylverbindung ist eine wichtige, teure Vorstufe bei der Synthese von racemischen 1,3-Dioxolan-4-on- bzw. 1,3-Oxathiolan-5-on-verbindungen. Sie wird, um Chemikalien und Kosten zu sparen, bevorzugt in die Synthese der 1,3-Dioxolan-4-one bzw. 1,3-Oxathiolan-5-one eingesetzt (s. Schema 1).

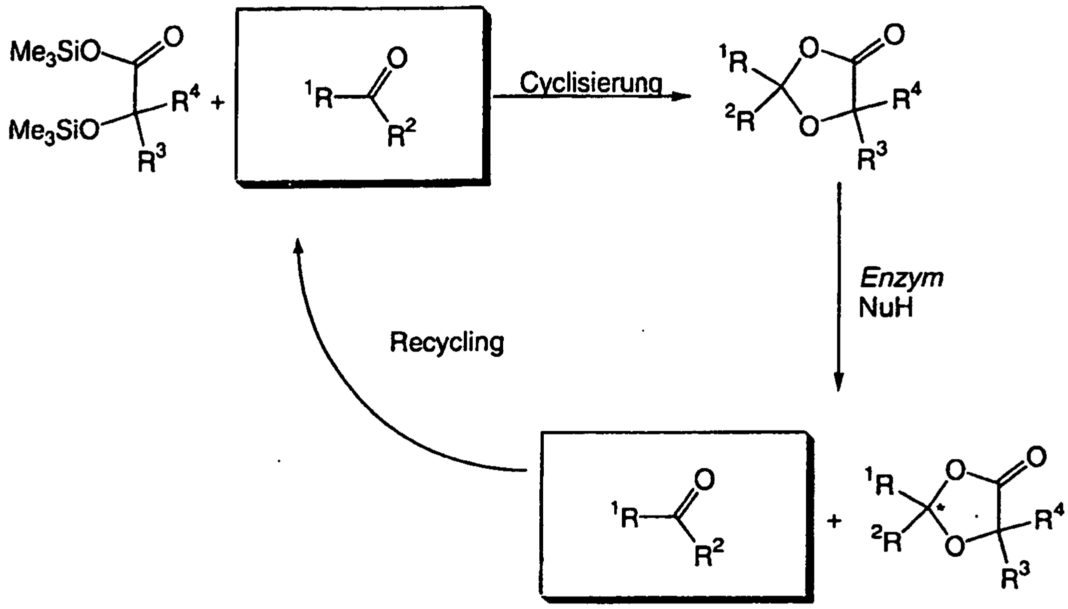


**Schema 1: Rückführung der bei der Racematspaltung entstehenden Carbonylverbindung am Beispiel von 1,3-Dioxolan-4-onen**

Die folgenden Beispiele dienen der weiteren Beschreibung der Erfindung.

Beispiel 1: (+) - (*R*)-2-Methylpropansäure (4-Oxo-1,3-dioxolan-2-yl)methylester (Batch-Prozess)

In einem 1 L 4-Halskolben werden 50.0 g (0.27 mol) racemischer 2-Methylpropansäure (4-Oxo-1,3-dioxolan-2-yl)methylester (X = O; $R^1$ = H; $R^2$ = $CH_2$-O-(CO)-$CH(CH_3)_2$; $R^3$, $R^4$ = H) in einer Mischung aus 185 mL MTBE und 185 mL Methanol (Nu = $OCH_3$) gelöst. Zu dieser Lösung gibt man 2.6 g Novozym® 435 und rührt die Mischung heftig.

An den 4-Halskolben ist über ein Bypass-System ein Polarimeter angeschlossen, mit dessen Hilfe der Reaktionsverlauf anhand der Messung des optischen Drehwerts der Lösung verfolgt wird. Bei erreichen der gewünschten Enantiomerenreinheit (Reaktionsverfolgung durch chiral-GC) wird zur Beendigung der Reaktion die Reaktionsmischung vom ungelösten Enzym abfiltriert. Anschließend wird die Reaktionsmischung im Vakuum eingeengt. Der Rückstand wird anschließend in 100 mL MTBE aufgenommen und 2 mal mit je 100 mL Wasser gewaschen. Die organische Phase wird über $Na_2SO_4$ getrocknet und dann i. Vak. vom Lösungsmittel befreit. Die Reinigung des Rohprodukts erfolgt durch Destillation.
Ausbeute: 10.3 g (0.05 mol; 20 %)
Sdp.: 55 °C (0.02 mbar)
$[\alpha]_D^{0}$ = +19.8 (neat); ee > 98 %

Beispiel 2: (+)-(*R*)-2-Methylpropansäure (4-Oxo-1,3-dioxolan-2-yl)methylester (Säulen-Prozess)

In einem thermostatisierten 0,6 L Glaskolben werden 50.0 g (0.27 mol) racemischer 2-Methylpropansäure (4-Oxo-1,3-dioxolan-2-yl)methylester (X = O; $R^1$ = H; $R^2$ = $CH_2$-O-(CO)-$CH(CH_3)_2$; $R^3$, $R^4$ = H) in einer Mischung aus 185 mL MTBE und 185 mL Methanol (Nu = $OCH_3$) gelöst. In eine separate Glassäule füllt man 1.3 g Novozym® 435 und pumpt über ein Schlauchsystem das Substrat-Lösungsmittelgemisch durch die Glassäule (Flußgeschwindigkeit 600 mL/h).
Zum Stoppen der Reaktion (nach - 25 h) wird das Umpumpen beendet, und das Rohprodukt wird wie in Beispiel 1 beschrieben gereinigt.

Beispiele 3-8:

Folgende Beispiele wurden entsprechend der Vorschrift in Beispiel 1 durchgeführt.

| | Substituenten entspr. Gleichung 5 | Racemat | Produkt | Selektivität nach Sih* |
|---|---|---|---|---|
| 3 | X = O; $R^1$ = H; $R^2$ = $-CH_2-C_6H_5$<br>$R^3$, $R^4$ = H | 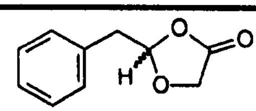 | (+)-2-Methylphenyl-1,3-dioxolan-4-on | 11 |
| 4 | X = O; $R^1$ = H; $R^2$ = $-C_6H_{11}$<br>$R^3$, $R^4$ = H | 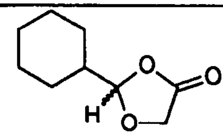 | (+)-2-Cyclohexyl-1,3-dioxolan-4-on | 65 |
| 5 | X = O; $R^1$ = H; $R^2$ = $-CH(CH_3)_2$<br>$R^3$, $R^4$ = H | 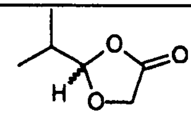 | (+)-2-iso-Propyl-1,3-dioxolan-4-on | 14 |
| 6 | X = O; $R^1$ = H; $R^2$ = $-C_7H_{15}$<br>$R^3$, $R^4$ = H | 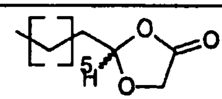 | (+)-2-Heptyl-1,3-dioxolan-4-on | 13 |
| 7 | X = S; $R^1$ = H; $R^2$ = $-C_6H_{11}$<br>$R^3$, $R^4$ = H | 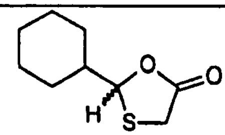 | (+)-2-Cyclohexyl-1,3-oxathiolan-5-on | 65 |
| 8 | X = S; $R^1$ = H; $R^2$ = $-CH_2O(CO)-CH(CH_3)_2$<br>$R^3$, $R^4$ = H | 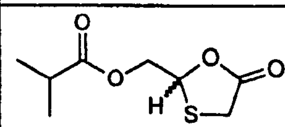 | (+)-Isobutyryloxymethyl-1,3-oxathiolan-5-on | 65 |

* Chen, C.-S. et al., J. Am. Chem. Soc. 104, 7294-7299 (1982)

## Patentansprüche

1. Verfahren zur Herstellung eines enantiomerenreinen 1,3-Dioxolan-4-on- bzw. 1,3-Oxathiolan-5-on-Derivats **dadurch gekennzeichnet, daß** ein Gemisch enthaltend enantiomere 1,3-Dioxolan-4-on- bzw. 1,3-Oxathiolan-5-on-Derivate und ein hydrolytisch wirksames Enzym in Gegenwart eines Nucleophils in Kontakt gebracht werden und der 1,3-Dioxolan-4-on- bzw. 1,3-Oxathiolan-5-on-ring eines Enantiomers durch das hydrolytisch wirksame Enzym gespalten wird und nach erfolgter Spaltung des einen Enantiomers das nicht gespaltene Enantiomer des 1,3-Dioxolan-4-on- bzw. 1,3-Oxathiolan-5-on-Derivats isoliert wird.

2. Verfahren gemäß Anspruch 1, **dadurch gekennzeichnet, dass** das Gemisch enthaltend enantiomere 1,3-Dioxolan-4-on bzw. 1,3-Oxathiolan-5-on-Derivate mittels eines Enzyms, das zur Spaltung einer Esterbindung befähigt ist in Gegenwart eines Nucleophils (NuH) wie in Gleichung dargestellt gespalten wird,

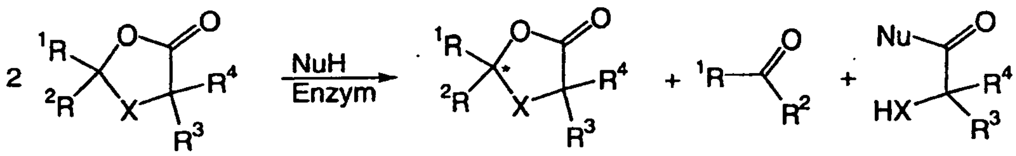


(Gleichung 5)

wobei X = Sauerstoff oder Schwefel und
die Reste $R^1$ und $R^2$ ungleich sind und unabhängig voneinander ausgewählt sind aus der Gruppe H, substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl, $CR^8R^9$-$O_n$- $(CO)_m$-$R^{10}$ und
die Reste $R^3$ und $R^4$ unabhängig voneinander ausgewählt sind aus der Gruppe H substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl oder die Reste $R^3$ und $R^4$ zusammen mit dem Kohlenstoff an den sie gebunden sind, ein unsubstituiertes oder substituiertes oder ein Heteroatom enthaltendes Cycloalkyliden bilden, und
Nu $OR^5$, $SR^5$, oder $NR^6R^7$ bedeutet wobei
der Rest $R^5$ ausgewählt ist aus der Gruppe H, substituiertes oder unsubstituiertes $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl und die Reste $R^6$ und $R^7$ unabhängig voneinander ausgewählt sind aus der Gruppe H, substituiertes oder unsubstituiertes $C_1$-$C_{18}$-Alkyl, $C_2$-$C_{18}$-Alkenyl, $C_2$-$C_{18}$-Alkinyl, $C_6$-$C_{18}$-Aryl, $C_3$-$C_{18}$-Heteroaryl, $C_6$-$C_{18}$-Aryl-$C_1$-$C_{18}$-Alkyl, $C_3$-$C_{18}$-Heteroaryl-$C_1$-$C_{18}$-Alkyl, $C_6$-$C_{18}$-Aryl-$C_2$-$C_{18}$-Alkenyl, $C_3$-$C_{18}$-Heteroaryl-$C_2$-$C_{18}$-Alkenyl und
die Reste $R^8$ und $R^9$ unabhängig voneinander ausgewählt sind aus der Gruppe substituiertes oder unsubstituiertes $C_6$-$C_{18}$-Aryl, $C_3$-$C_{18}$-Heteroaryl, $C_1$-$C_{18}$-Alkyl, $C_2$-$C_{18}$-Alkenyl, $C_2$-$C_{18}$-Alkinyl, $C_6$-$C_{18}$-Aryl-$C_1$-$C_{18}$-Alkyl, $C_3$-$C_{18}$-Heteroaryl-$C_1$-$C_{18}$-Alkyl, , $C_6$-$C_{18}$-Aryl-$C_2$-$C_{18}$-Alkenyl, $C_3$-$C_{18}$-Heteroaryl-$C_2$-$C_{18}$-Alkenyl, $C_1$-$C_{18}$-Alkoxy-$C_1$-$C_{18}$-Alkyl, $C_1$-$C_{18}$-Alkoxy-$C_2$-$C_{18}$-Alkenyl, $C_6$-$C_{18}$-Aryloxy-$C_1$-$C_{18}$-Alkyl, $C_6$-$C_{18}$-Aryloxy-$C_2$-$C_{18}$-Alkenyl, $C_3$-$C_8$-Cycloalkyl, $C_3$-$C_8$-Cycloalkyl-$C_1$-$C_{18}$-Alkyl, $C_3$-$C_8$-Cycloalkyl-$C_2$-$C_{18}$-Alkenyl oder die Reste $R^8$ und $R^9$ zusammen mit dem Kohlenstoff an den sie gebunden sind, ein unsubstituiertes oder substituiertes oder ein Heteroatom enthaltendes Cycloalkyliden bilden, und
m und n unabhängig voneinander 0 oder 1 bedeuten, und für den Rest $R^{10}$ gilt:

wenn m = 0 dann ist Rest $R^{10}$ ausgewählt aus der Gruppe substituiertes oder unsubstituiertes, $C_1$-$C_{18}$-Alkyl, $C_2$-$C_{18}$-Alkenyl oder $C_2$-$C_{18}$-Alkinyl, substituiertes oder unsubstituiertes $C_6$-$C_{18}$-Aryl, $C_3$-$C_{18}$-Heteroaryl, substituiertes oder unsubstituiertes Silaalkyl oder Silaaryl, und
wenn m = 1 dann ist Rest $R^{10}$ ausgewählt aus der Gruppe substituiertes oder unsubstituiertes Aryl, substituiertes oder unsubstituiertes, $C_1$-$C_{18}$-Alkyl, $C_2$-$C_{18}$-Alkenyl oder $C_2$-$C_{18}$-Alkinyl.

**3.** Verfahren gemäß Anspruch 1 oder 2, **dadurch gekennzeichnet, dass** das hydrolytisch wirksame Enzym eine Lipase oder Esterase ist.

**4.** Verfahren gemäß einem der Ansprüche 1 bis 3, **dadurch gekennzeichnet, dass** das Enzym direkt oder als Immobilisat eingesetzt wird.

**5.** Verfahren gemäß einem der Ansprüche 1 bis 4, **dadurch gekennzeichnet, dass** Enzym zu Dioxolanon/Oxathiolanon-Derivat berechnet als Molverhältnis zwischen Enzym und Dioxolanon/Oxathiolanon-Derivat in einem Verhältnis von 1 : 1000 bis 1 : 50000000 liegt.

**6.** Verfahren gemäß einem der Ansprüche 1 bis 5, **dadurch gekennzeichnet, dass** das Nucleophil ein sauerstoffhaltiges Nucleophil ist.

**7.** Verfahren gemäß Anspruch 6, **dadurch gekennzeichnet, dass** das sauerstoffhaltige Nucleophil ein niederer unverzweigter Alkohol oder Wasser ist.

**8.** Verfahren gemäß Anspruch 7, **dadurch gekennzeichnet, dass** der niedere unverzweigte Alkohol Methanol oder Ethanol ist.

**9.** Verfahren gemäß einem der Ansprüche 1 bis 8, **dadurch gekennzeichnet, dass** es in Gegenwart eines Cosolvens durchgeführt wird.

**10.** Verfahren gemäß Anspruch 9, **dadurch gekennzeichnet, dass** das Cosolvens ausgewählt ist aus der Gruppe aliphatische Kohlenwasserstoffe, aromatische Kohlenwasserstoffe, halogenierte Kohlenwasserstoffe, Ether, Alkohole, Ester oder Acetonitril oder Mischungen der genannten Verbindungen.

**11.** Verfahren gemäß einem der Ansprüche 1 bis 10, **dadurch gekennzeichnet, dass** die Reaktion bei Temperaturen zwischen 0 und 75 °C durchgeführt wird.

**12.** Verfahren gemäß einem der Ansprüche 1 bis 11, **dadurch gekennzeichnet, dass** die Reaktion zwischen 10 Minuten und bis zu 7 Tagen durchgeführt wird.

**13.** Verfahren gemäß einem der Ansprüche 1 bis 12, **dadurch gekennzeichnet, dass** die Isolierung des nichtgespaltenen Enantiomers durch Abtrennung der bei der Reaktion entstandenen Nebenprodukte und des Lösungsmittels erfolgt.

**14.** Verfahren nach Anspruch 13 **dadurch gekennzeichnet, daß** die Nebenprodukte durch Extraktion und Destillation abgetrennt werden.

## Claims

**1.** Process for preparing an enantiopure 1,3-dioxolan-4-one or 1,3-oxathiolan-5-one derivative, **characterized in that** a mixture containing enantiomeric 1,3-dioxolan-4-one or 1,3-oxathiolan-5-one derivatives and an enzyme with hydrolytic activity are brought into contact in the presence of a nucleophile, and the 1,3-dioxolan-4-one or 1,3-oxathiolan-5-one ring of one enantiomer is cleaved by the enzyme with hydrolytic activity and, after the cleavage of one enantiomer has taken place, the uncleaved enantiomer of the 1,3-dioxolan-4-one or 1,3-oxathiolan-5-one derivative is isolated.

**2.** Process according to Claim 1, **characterized in that** the mixture containing enantiomeric 1,3-dioxolan-4-one or 1,3-oxathiolan-5-one derivatives is cleaved by means of an enzyme which is able to cleave an ester linkage in the presence of a nucleophile (NuH) as depicted in the equation,

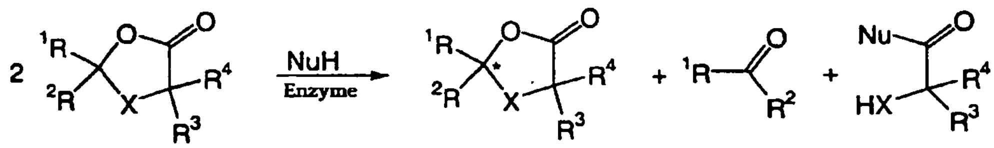


(Equation 5)

where X = oxygen or sulphur and
the radicals $R^1$ and $R^2$ are different and are selected independently of one another from the group of H, substituted or unsubstituted $C_6$-$C_{18}$-aryl, $C_3$-$C_{18}$-heteroaryl, $C_1$-$C_{18}$-alkyl, $C_2$-$C_{18}$-alkenyl, $C_2$-$C_{18}$-alkynyl, $C_6$-$C_{18}$-aryl-$C_1$-$C_{18}$-alkyl, $C_3$-$C_{18}$-heteroaryl-$C_1$-$C_{18}$-alkyl, $C_6$-$C_{18}$-aryl-$C_2$-$C_{18}$-alkenyl, $C_3$-$C_{18}$-heteroaryl-$C_2$-$C_{18}$-alkenyl, $C_1$-$C_{18}$-alkoxy-$C_1$-$C_{18}$-alkyl, $C_1$-$C_{18}$-alkoxy $C_2$-$C_{18}$-alkenyl, $C_6$-$C_{18}$-aryloxy-$C_1$-$C_{18}$-alkyl, $C_6$-$C_{18}$-aryloxy-$C_2$-$C_{18}$-alkenyl, $C_3$-$C_8$-cycloalkyl, $C_3$-$C_8$-cycloalkyl-$C_1$-$C_{18}$-alkyl, $C_3$-$C_8$-cycloalkyl-$C_2$-$C_{18}$-alkenyl, $CR^8R^9$-$O_n$- $(CO)_m$-$R^{10}$ and
the radicals $R^3$ and $R^4$ are selected independently of one another from the group H of substituted or unsubstituted $C_6$-$C_{18}$-aryl, $C_3$-$C_{18}$-heteroaryl, $C_1$-$C_{18}$-alkyl, $C_2$-$C_{18}$-alkenyl, $C_2$-$C_{18}$-alkynyl, $C_6$-$C_{18}$-aryl-$C_1$-$C_{18}$-alkyl, $C_3$-$C_{18}$-heteroaryl-$C_1$-$C_{18}$-alkyl, $C_6$-$C_{18}$-aryl-$C_2$-$C_{18}$-alkenyl, $C_3$-$C_{18}$-heteroaryl-$C_2$-$C_{18}$-alkenyl, $C_1$-$C_{18}$-alkoxy-$C_1$-$C_{18}$-alkyl, $C_1$-$C_{18}$-alkoxy-$C_2$-$C_{18}$-alkenyl, $C_6$-$C_{18}$-aryloxy-$C_1$-$C_{18}$-alkyl, $C_6$-$C_{18}$-aryloxy-$C_2$-$C_{18}$-alkenyl, $C_3$-$C_8$-cycloalkyl, $C_3$-$C_8$-cycloalkyl-$C_1$-$C_{18}$-alkyl, $C_3$-$C_8$-cycloalkyl-$C_2$-$C_{18}$-alkenyl or
the radicals $R^3$ and $R^4$ form, together with the carbon to which they are bonded, an unsubstituted or substituted or a heteroatom-containing cycloalkylidene and
Nu is $OR^5$, $SR^5$ or $NR^6R^7$, where
the radical $R^5$ is selected from the group of H, substituted or unsubstituted $C_1$-$C_{18}$-alkyl, $C_2$-$C_{18}$-alkenyl, $C_2$-$C_{18}$-alkynyl, $C_6$-$C_{18}$-aryl-$C_1$-$C_{18}$-alkyl, $C_3$-$C_{18}$-heteroaryl-$C_1$-$C_{18}$-alkyl, $C_6$-$C_{18}$-aryl-$C_2$-$C_{18}$-alkenyl, $C_3$-$C_{18}$-heteroaryl-$C_2$-$C_{18}$-alkenyl, and
the radicals $R^6$ and $R^7$ are selected independently of one another from the group of H, substituted or unsubstituted $C_1$-$C_{18}$-alkyl, $C_2$-$C_{18}$-alkenyl, $C_2$-$C_{18}$-alkynyl, $C_6$-$C_{18}$-aryl, $C_3$-$C_{18}$-heteroaryl, $C_6$-$C_{18}$-aryl-$C_1$-$C_{18}$-alkyl, $C_3$-$C_{18}$-heteroaryl-$C_1$-$C_{18}$-alkyl, $C_6$-$C_{18}$-aryl-$C_2$-$C_{18}$-alkenyl, $C_3$-$C_{18}$-heteroaryl-$C_2$-$C_{18}$-alkenyl and the radicals $R^8$ and $R^9$ are selected independently of one another from the group of substituted or unsubstituted $C_6$-$C_{18}$-aryl, $C_3$-$C_{18}$-heteroaryl, $C_1$-$C_{18}$-alkyl, $C_2$-$C_{18}$-alkenyl, $C_2$-$C_{18}$-alkynyl, $C_6$-$C_{18}$-aryl-$C_1$-$C_{18}$-alkyl, $C_3$-$C_{18}$-heteroaryl-$C_1$-$C_{18}$-alkyl, $C_6$-$C_{18}$-aryl-$C_2$-$C_{18}$-alkenyl, $C_3$-$C_{18}$-heteroaryl-$C_2$-$C_{18}$-alkenyl, $C_1$-$C_{18}$-alkoxy-$C_1$-$C_{18}$-alkyl, $C_1$-$C_{18}$-alkoxy-$C_2$-$C_{18}$-alkenyl, $C_6$-$C_{18}$-aryloxy-$C_1$-$C_{18}$-alkyl, $C_6$-$C_{18}$-aryloxy-$C_2$-$C_{18}$-alkenyl, $C_3$-$C_8$-cycloalkyl, $C_3$-$C_8$-cycloalkyl-$C_1$-$C_{18}$-alkyl, $C_3$-$C_8$-cycloalkyl-$C_2$-$C_{18}$-alkenyl or
the radicals $R^8$ and $R^9$ form, together with the carbon to which they are bonded, an unsubstituted or substituted or a heteroatom-containing cycloalkylidene, and
m and n are, independently of one another, 0 or 1, and the following applies to the radical $R^{10}$:

if m is 0 then the radical $R^{10}$ is selected from the group of substituted or unsubstituted $C_1$-$C_{18}$-alkyl, $C_2$-$C_{18}$-alkenyl or $C_2$-$C_{18}$-alkynyl, substituted or unsubstituted $C_6$-$C_{18}$-aryl, $C_3$-$C_{18}$-heteroaryl, substituted or unsubstituted silaalkyl or silaaryl, and
if m is 1 then the radical $R^{10}$ is selected from the group of substituted or unsubstituted aryl, substituted or unsubstituted $C_1$-$C_{18}$-alkyl, $C_2$-$C_{18}$-alkenyl or $C_2$-$C_{18}$-alkynyl.

3. Process according to Claim 1 or 2, **characterized in that** the enzyme with hydrolytic activity is a lipase or esterase.

4. Process according to any of Claims 1 to 3, **characterized in that** the enzyme is employed directly or in immobilized form.

5. Process according to any of Claims 1 to 4, **characterized in that** the enzyme to dioxolanone/oxathiolanone derivative ratio, calculated as molar ratio between enzyme and dioxolanone/oxathiolanone derivative, is from 1:1 000 to 1:50 000 000.

6. Process according to any of Claims 1 to 5, **characterized in that** the nucleophile is an oxygen-containing nucleophile.

7. Process according to Claim 6, **characterized in that** the oxygen-containing nucleophile is a lower unbranched alcohol or water.

**8.** Process according to Claim 7, **characterized in that** the lower unbranched alcohol is methanol or ethanol.

**9.** Process according to any of Claims 1 to 8, which is carried out in the presence of a cosolvent.

**10.** Process according to Claim 9, **characterized in that** the cosolvent is selected from the group of aliphatic hydrocarbons, aromatic hydrocarbons, halogenated hydrocarbons, ethers, alcohols, esters or acetonitrile or mixtures of said compounds.

**11.** Process according to any of Claims 1 to 10, **characterized in that** the reaction is carried out at temperatures between 0 and 75°C.

**12.** Process according to any of Claims 1 to 11, **characterized in that** the reaction is carried out for between 10 minutes and up to 7 days.

**13.** Process according to any of Claims 1 to 12, **characterized in that** the uncleaved enantiomer is isolated by removing the byproducts of the reaction and the solvent.

**14.** Process according to Claim 13, **characterized in that** the byproducts are removed by extraction and distillation.

## Revendications

**1.** Procédé de préparation d'un dérivé 1,3-dioxolan-4-one ou 1,3-oxathiolan-5-one énantiomériquement pur, **caractérisé en ce qu'**on met en contact un mélange contenant des dérivés 1,3-dioxolan-4-one ou 1,3-oxathiolan-5-one énantiomères et un enzyme à effet hydrolytique en présence d'un nucléophile, la séparation du noyau 1,3-dioxolan-4-one ou 1,3-oxathiolan-5-one d'un énantiomère étant provoquée par l'enzyme à effet hydrolytique, et **en ce qu'**on isole, une fois terminée la séparation dudit énantiomère, l'énantiomère non séparé du dérivé 1,3-dioxolan-4-one ou 1,3-oxathiolan-5-one.

**2.** Procédé selon la revendication 1, **caractérisé en ce qu'**on sépare le mélange contenant les dérivés 1,3-dioxalan-4-one ou 1,3-oxathiolan-5-one énantiomères au moyen d'un enzyme qui est capable de couper une liaison ester en présence d'un nucléophile (NuH) comme représenté dans l'équation :

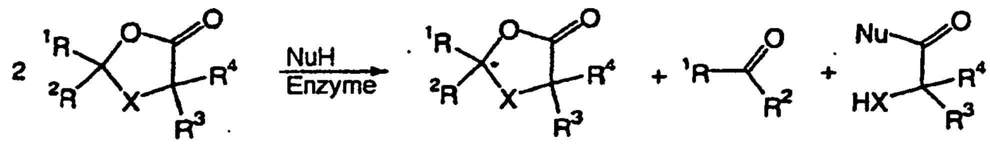


(Equation 5)

dans laquelle X = oxygène ou soufre et
les radicaux $R^1$ et $R^2$ sont différents ec choisis indépendamment l'un de l'autre dans le groupe H, aryle en $C_6$-$C_{18}$, hétéroaryle en $C_3$-$C_{18}$, alkyle en $C_1$-$C_{18}$, alcényle en $C_2$-$C_{18}$, alcinyle en $C_2$-$C_{18}$, aryle en $C_6$-$C_{18}$-alkyle en $C_1$-$C_{18}$, hétéroaryle en $C_3$-$C_{18}$-alkyle en $C_1$-$C_{18}$, aryle en $C_6$-$C_{18}$-alcényle en $C_2$-$C_{18}$, hétéroaryle en $C_3$-$C_{18}$-alcényle en $C_2$-$C_{18}$, alcoxy en $C_1$-$C_{18}$-alkyle en $C_1$-$C_{18}$ , alcoxy en $C_1$-$C_{18}$-alcényle en $C_2$-$C_{18}$, aryloxy en $C_6$-$C_{18}$-alkyle en $C_1$-$C_{18}$, aryloxy en $C_6$-$C_{18}$-alcényle en $C_2$-$C_{18}$, cycloalkyle en $C_3$-$C_8$, cycloalkyle en $C_3$-$C_8$-alkyle en $C_1$-$C_{18}$, cycloalkyle en $C_3$-$C_8$-alcényle en $C_2$-$C_{18}$, $CR^8R^9$-$O_n$- $(CO)_m$-$R^{10}$, substitués ou non substitués, et
les radicaux $R^3$ et $R^4$ sont choisis indépendamment l'un de l'autre dans le groupe H aryle en $C_6$-$C_{18}$, hétéroaryle en $C_3$-$C_{18}$, alkyle en $C_1$-$C_{18}$, alcényle en $C_2$-$C_{18}$, alcinyle en $C_2$-$C_{18}$, aryle en $C_6$-$C_{18}$-alkyle en $C_1$-$C_{18}$, hétéroaryle en $C_3$-$C_{18}$-alkyle en $C_1$-$C_{18}$, aryle en $C_6$-$C_{18}$-alcényle en $C_2$-$C_{18}$, hétéroaryle en $C_3$-$C_{18}$-alcényle en $C_2$-$C_{18}$, alcoxy en $C_1$-$C_{18}$-alkyle en $C_1$-$C_{18}$, alcoxy en $C_1$-$C_{18}$-alcényle en $C_2$-$C_{18}$, aryloxy en $C_6$-$C_{18}$-alkyle en $C_1$-$C_{18}$, aryloxy en $C_6$-$C_{18}$-alcényle en $C_2$-$C_{18}$, cycloalkyle en $C_3$-$C_8$, cycloalkyle en $C_3$-$C_8$-alkyle en $C_1$-$C_{18}$, cycloalkyle en $C_3$-$C_8$-alcényle en $C_2$-$C_{18}$, substitués ou non substitués, ou les radicaux $R^3$ et $R^4$ forment avec l'atome de carbone auquel ils sont fixés un cycloalkylidène substitué ou non substitué ou contenant un hétéroatome, et
Nu représente $OR^5$, $SR^5$ ou $NR^6R^7$, le radical $R^5$ étant choisi dans le groupe H, alkyle en $C_1$-$C_{18}$, alcényle en $C_2$-$C_{18}$, alcinyle en $C_2$-$C_{18}$, aryle en $C_6$-$C_{18}$-alkyle en $C_1$-$C_{18}$, hétéroaryle en $C_3$-$C_{18}$-alkyle en $C_1$-$C_{18}$, aryle en

$C_6$-$C_{18}$-alcényle en $C_2$-$C_{18}$, hétéroaryle en $C_3$-$C_{18}$-alcényle en $C_2$-$C_{18}$, substitués ou non substitués, et les radicaux $R^6$ et $R^7$ sont choisis indépendamment l'un de l'autre dans le groupe H, alkyle en $C_1$-$C_{18}$, alcényle en $C_2$-$C_{18}$, alcinyle en $C_2$-$C_{18}$, aryle en $C_6$-$C_{18}$, hétéroaryle en $C_3$-$C_{18}$, aryle en $C_6$-$C_{18}$-alkyle en $C_1$ -$C_{18}$, hétéroaryle en $C_3$-$C_{18}$-alkyle en $C_1$-$C_{18}$, aryle en $C_6$-$C_{18}$-alcényle en $C_2$-$C_{18}$, hétéroaryle en $C_3$-$C_{18}$-alcényle en $C_2$-$C_{18}$, substitués ou non substitués, et les radicaux $R^8$ et $R^9$ sont choisis indépendamment l'un de l'autre dans le groupe aryle en $C_6$-$C_{18}$, hétéroaryle en $C_3$-$C_{18}$, alkyle en $C_1$-$C_{18}$, alcényle en $C_2$-$C_{18}$, alcinyle en $C_2$-$C_{18}$, aryle en $C_6$-$C_{18}$-alkyle en $C_1$-$C_{18}$, hétéroaryle en $C_3$-$C_{18}$-alkyle en $C_1$-$C_{18}$, aryle en $C_6$-$C_{18}$-alcényle en $C_2$-$C_{18}$, hétéroaryle en $C_3$-$C_{18}$-alcényle en $C_2$-$C_{18}$, alcoxy en $C_1$-$C_{18}$-alkyle en Ci-$C_{18}$, alcoxy en $C_1$-$C_{18}$-alcényle en $C_2$-$C_{18}$, aryloxy en $C_6$-$C_{18}$-alkyle en $C_1$-$C_{18}$, aryloxy en $C_6$-$C_{18}$-alcényle en $C_2$-$C_{18}$, cycloalkyle en $C_3$-$C_8$, cycloalkyle en $C_3$-$C_8$-alkyle en $C_1$-$C_{18}$, cycloalkyle en $C_3$-$C_8$-alcényle en $C_2$-$C_{18}$, substitués ou non substitués, ou les radicaux $R^8$ et $R^9$ forment avec l'atome de carbone auquel ils sont fixés un cycloalkylidène non substitué ou substitué ou contenant un hétéroatome et
m et n ont pour valeur, indépendamment l'un de l'autre, 0 ou 1, et pour le radical $R^{10}$ :

lorsque m = 0 , le radical $R^{10}$ est choisi dans le groupe alkyle en $C_1$-$C_{18}$, alcényle en $C_2$-$C_{18}$ ou alcinyle en $C_2$-$C_{18}$, substitué ou non substitué, aryle en $C_6$-$C_{18}$, hétéroaryle en $C_3$-$C_{18}$, substitué ou non substitué, silaalkyle ou silaaryle substitué ou non substitué, et
lorsque m = 1, le radical $R^{10}$ est choisi dans le groupe aryle substitué ou non substitué, alkyle en $C_1$-$C_{18}$, alcényle en $C_2$-$C_{18}$ ou alcinyle en $C_2$-$C_{18}$, substitué ou non substitué.

3. Procédé selon la revendication 1 ou 2, **caractérisé en ce que** l'enzyme à effet hydrolytique est une lipase ou une estérase.

4. Procédé selon l'une quelconque des revendications 1 à 3, **caractérisé en ce que** l'enzyme est utilisé directement ou sous forme d'immobilisat.

5. Procédé selon l'une quelconque des revendications 1 à 4, **caractérisé en ce que** le rapport de l'enzyme au dérivé dioxolanone/oxathiolanone, calculé en tant que rapport molaire entre l'enzyme et le dérivé dioxolanone/oxathiolanone, se trouve dans un rapport de 1:1 000 à 1:50 000 000.

6. Procédé selon l'une quelconque des revendications 1 à 5, **caractérisé en ce que** le nucléophile est un nucléophile contenant de l'oxygène.

7. Procédé selon la revendication 6, **caractérisé en ce que** le nucléophile contenant de l'oxygène est un alcool non ramifié inférieur ou l'eau.

8. Procédé selon la revendication 7, **caractérisé en ce que** l'alcool non ramifié inférieur est le méthanol ou l'éthanol.

9. Procédé selon l'une quelconque des revendications 1 à 8, **caractérisé en ce qu'**il est mis en oeuvre en présence d'un cosolvant.

10. Procédé selon la revendication 9, **caractérisé en ce que** le cosolvant est choisi dans le groupe des hydrocarbures aliphatiques, hydrocarbures aromatiques, hydrocarbures halogénés, éthers, alcools, esters ou acétonitrile ou des mélanges des composés cités.

11. Procédé selon l'une quelconque des revendications 1 à 10, **caractérisé en ce que** la réaction est effectuée à des températures situées entre 0 et 75°C.

12. Procédé selon l'une quelconque des revendications 1 à 11, **caractérisé en ce que** la réaction est effectuée dans un temps situé entre 10 minutes et 7 jours.

13. Procédé selon l'une quelconque des revendications 1 à 12, **caractérisé en ce que** l'isolement de l'énantiomère non séparé a lieu par séparation des sous-produits formés lors de la réaction et du solvant.

14. Procédé selon la revendication 13, **caractérisé en ce que** les sous-produits sont séparés par extraction et distillation.